# EUROPEAN PATENT APPLICATION

(11) **EP 3 597 235 A1**
(43) Date of publication of application: **22.01.2020**
(21) Application number: 18767406.4
(22) Date of filing: 15.03.2018
(51) Int. Cl.: A61M 5/20, A61M 5/142

(54) **MEDICINAL LIQUID ADMINISTERING DEVICE**

(30) Priority: 16.03.2017 JP 2017050739
(71) Applicant: Terumo Kabushiki Kaisha, Tokyo 151-0072 (JP)
(72) Inventor: KONDO, Akira, Ashigarakami-gun Kanagawa 259-0151 (JP)
(74) Representative: Casalonga
(86) International application number: PCT/JP2018/010148
(87) International publication number: WO 2018/168988

(57) **Abstract**

A liquid medication administration device (10) includes a cylindrical body (12), a gasket (34), a plunger body (36) that pushes a liquid medication (M) out of the cylindrical body (12), an advancing mechanism (38) that advances the plunger body (36), a drive mechanism (39) that drives the advancing mechanism (38), a chassis member (24) that supports at least the cylindrical body (12) and the advancing mechanism (38), and a housing (28) that houses the cylindrical body (12), the plunger body (36), the advancing mechanism (38), the drive mechanism (39), and the chassis member (24). The chassis member (24) includes a first supporting portion that supports a distal surface of a first supported portion of the cylindrical body (12) and a second supporting portion that supports a proximal surface of a second supported portion of the advancing mechanism (38).

## Description

### Technical Field

The present invention relates to a liquid medication administration device for pushing a liquid medication out of a cylindrical body by a plunger mechanism and for administering the liquid medication to a living body.

### Background Art

In the related art, there is known a syringe-pump type liquid medication administration device for administrating a liquid medication filled in a cylindrical body to a living body by a pressing force of a plunger. This type of liquid medication administration device includes a barrel type cylindrical body and a plunger mechanism that pushes the liquid medication out of the cylindrical body (see, for example, JP 9-294807 A). The cylindrical body and the plunger mechanism are housed inside a housing and supported by the housing.

### Summary of Invention

When a liquid medication to be used has a high viscosity, a liquid medication administration device in the related art requires a large force to push the liquid medication out of a cylindrical body. Accordingly, the large force acts on a housing when the liquid medication is pushed out, leading to deformation (warpage) of the housing. Deformation of the housing causes an inconstant moving speed of a plunger mechanism, which may reduce the accuracy of liquid feeding speed of the liquid medication.

The present invention has been made in consideration of such problems, and an object of the present invention is to provide a liquid medication administration device that prevents deformation of a housing when a liquid medication is pushed out.

In order to achieve the object, a liquid medication administration device according to an embodiment of the present invention is for administering a liquid medication to a living body, the liquid medication administration device including: a cylindrical body filled with the liquid medication; a gasket slidably disposed inside the cylindrical body; a plunger body that pushes the gasket in a distal direction to push the liquid medication out of the cylindrical body; an advancing mechanism that advances the plunger body in the distal direction; a drive mechanism that drives the advancing mechanism to advance the plunger body in the distal direction; a chassis member that supports at least the cylindrical body and the advancing mechanism; and a housing that houses the cylindrical body, the plunger body, the advancing mechanism, the drive mechanism, and the chassis member, wherein the cylindrical body includes a first supported portion supported by the chassis member, the advancing mechanism includes a second supported portion supported by the chassis member, the chassis member includes a base plate that has a board shape and has an upper surface and a lower surface, a first supporting portion that protrudes from the upper surface of the base plate and supports at least a distal surface of the first supported portion of the cylindrical body, and a second supporting portion that protrudes from the upper surface of the base plate and supports at least a proximal surface of the second supported portion of the advancing mechanism, and the housing includes a chassis supporting portion that extends at least along a lower surface of the base plate and supports the base plate.

According to the liquid medication administration device of the present invention having the arrangement, the chassis member integrates the cylindrical body and the advancing mechanism, and the chassis member is fixed to the housing. With such an arrangement, the housing is reinforced by the chassis member, and a dual structure formed by the chassis member and the housing prevents deformation of the housing when the liquid medication is pushed out. Accordingly, an axis of the advancing mechanism is prevented from being inclined relative to an axis of the cylindrical body, which enables administration of the liquid medication at a desired liquid feeding speed.

The chassis member may include a rib structure protruding from the base plate and extending along an axial direction of the cylindrical body.

This arrangement efficiently enhances rigidity of the chassis member and prevents warpage.

The rib structure may extend at least from a portion near the first supporting portion to a portion near the second supporting portion.

This arrangement more efficiently prevents the chassis member from deforming in a direction in which the axis of the advancing mechanism inclines relative to the axis of the cylindrical body.

The rib structure may include an edge rib disposed at an edge of the base plate along the axial direction of the cylindrical body.

This arrangement enables the rib to be disposed without interfering with the plunger mechanism.

The edge rib may include a hole, and the chassis member may be fixed to the housing by a fastening component inserted into the hole.

According to this arrangement, the edge rib doubles as a boss into which the fastening component is inserted. Due to the fastening component, it is possible to simplify the arrangement of the chassis member fixed to the housing.

The rib structure may be continuous with the first supporting portion.

In this arrangement, the first supporting portion is continuous with the rib structure, which efficiently enhances rigidity of the chassis member.

The rib structure may include a lower surface rib protruding from the lower surface of the base plate.

This arrangement efficiently enhances rigidity of the chassis member while avoiding an interference between the advancing mechanism and the rib structure.

The chassis supporting portion may include a slit into which the lower surface rib is inserted.

This arrangement enables positioning of the chassis member relative to the housing before fixing the chassis member by the fastening member. Furthermore, the lower surface rib is inserted into the slit, which reduces the device in thickness.

The rib structure and the first supporting portion may be formed in an integrated manner with the base plate.

This arrangement makes the chassis member less likely to warp.

The cylindrical body may include a barrel filled with the liquid medication and a flange protruding outward from a proximal end of the barrel. The first supporting portion may support a distal surface of the flange and a proximal surface of the flange.

This arrangement enables independent positioning of the cylindrical body on the chassis member, which facilitates assembly.

The second supporting portion may be disposed on a metallic contact member attached to the base plate.

Making the contact member from metal prevents the second supporting portion itself from bending and being unable to support the second supported portion.

The advancing mechanism includes: a feed screw extending along the axial direction of the cylindrical body and having an outer periphery provided with an external thread; a female screw disposed on the plunger body and having an inner periphery provided with an internal thread that is screwed into the external thread; and a guide rail that extends along the axial direction of the cylindrical body and supports the female screw; wherein the female screw is supported by the guide rail, being non-rotatable with respect to the chassis member and movable with respect to the chassis member along the axial direction of the cylindrical body, the feed screw is rotatably supported by the chassis member, the drive mechanism is configured to rotate the feed screw, and the female screw advances in the distal direction with respect to the feed screw along with rotation of the feed screw.

The feed screw extends in the axial direction, which increases the loss of force when the housing is warped. Therefore, it is more important to prevent warpage.

The drive mechanism may include a motor and a driving gear fixed to an output shaft of the motor. The feed screw may include a feed screw shaft extending along the axial direction of the cylindrical body and including the external thread and may include a driven gear connected to the feed screw shaft in a non-rotatable manner and to which rotation of the driving gear is transmitted.

The driven gear may include a gear body having an outer periphery provided with a plurality of teeth and include a rotary shaft disposed at a rotation center of the gear body and protruding from the gear body in both the distal direction and the opposite direction, or a proximal direction. The rotary shaft may have a distal end connected to a proximal end of the feed screw shaft, and the proximal end of the rotary shaft may be supported by the second supporting portion.

According to the liquid medication administration device of the present invention, it is possible to prevent the housing from being deformed when the liquid medication is pushed out.

### Brief Description of Drawings

Fig. 1 is an appearance view of a liquid medication administration device according to an embodiment of the present invention.
Fig. 2 is an exploded perspective view of the liquid medication administration device.
Fig. 3 is a perspective view of a liquid feeding unit.
Fig. 4 is a perspective view for describing a nut member and peripheral structures thereof.
Fig. 5 is a perspective view of a chassis member seen from the front lower side.
Fig. 6 is a perspective view of the liquid feeding unit when liquid feeding is completed.
Fig. 7 is a cross-sectional perspective view for describing how a switch detects the completion of liquid feeding.

### Description of Embodiments

A preferred embodiment of a liquid medication administration device 10 according to the present invention will now be described with reference to the accompanying drawings.

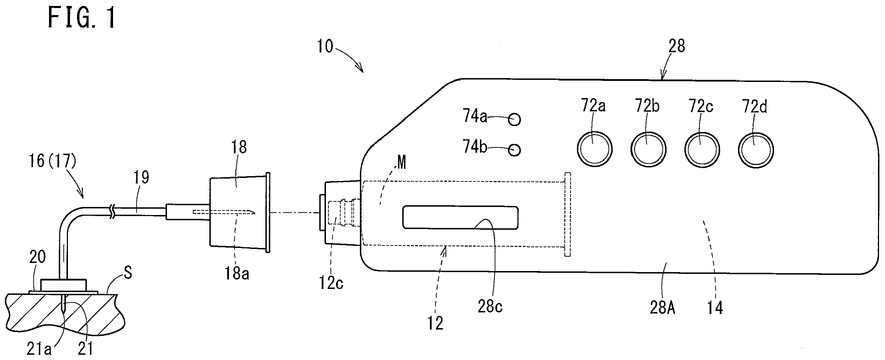

The liquid medication administration device 10 according to this embodiment illustrated in Fig. 1 is used to administer a liquid medication M to a living body. Taking a relatively long time (for example, several minutes to several hours), the liquid medication administration device 10 continuously administers the liquid medication M filled in a cylindrical body 12 to the living body by a pressing force of a plunger mechanism 14. The liquid medication administration device 10 may administer the liquid medication M to the living body at intervals . Examples of the liquid medication M include protein formulations, narcotic analgesics, and diuretics.

As illustrated in Fig. 1, when the liquid medication administration device 10 is in use, an administration line 16 or, for example, a patch type needle tube 17, is connected to the liquid medication administration device 10, and the liquid medication M discharged from the cylindrical body 12 is injected into the body of a patient through the needle tube 17. The needle tube 17 is provided with a connector 18, a liquid feeding tube 19, a patch 20, and a needle 21. The connector 18 is connectable to a distal end 12c of the cylindrical body 12. The liquid feeding tube 19 is flexible and has one end connected to the connector 18. The patch 20 is connected to the other end of the liquid feeding tube 19 and is to be stuck to skin S. The needle 21 protrudes from the patch 20. The needle 21 is inserted into the skin S in a substantially perpendicular manner. The needle 21 may be inserted into the skin S in an oblique manner.

The administration line 16 connected to the liquid medication administration device 10 is not limited to the patch type needle tube 17 and may be, for example, one in which a puncture needle (such as a butterfly needle) is connected to a distal end of the liquid feeding tube 19. Alternatively, the administration line 16 may be a bent needle connectable to the distal end 12c of the cylindrical body 12 without the liquid feeding tube 19 being involved. In this case, the bent needle is bent, for example, at about 90 degrees downward from the distal end 12c of the cylindrical body 12 and is perpendicularly inserted into the skin S when the liquid medication administration device 10 is fixed (stuck) to the skin S. Furthermore, the distal end 12c of the cylindrical body 12, the administration line 16, and a part of the needle may be disposed inside the cylindrical body 12, and a needle tip may protrude from the cylindrical body 12. Even in this case, the needle 21 is perpendicularly inserted into the skin S when the liquid medication administration device 10 is fixed (stuck) to the skin S.

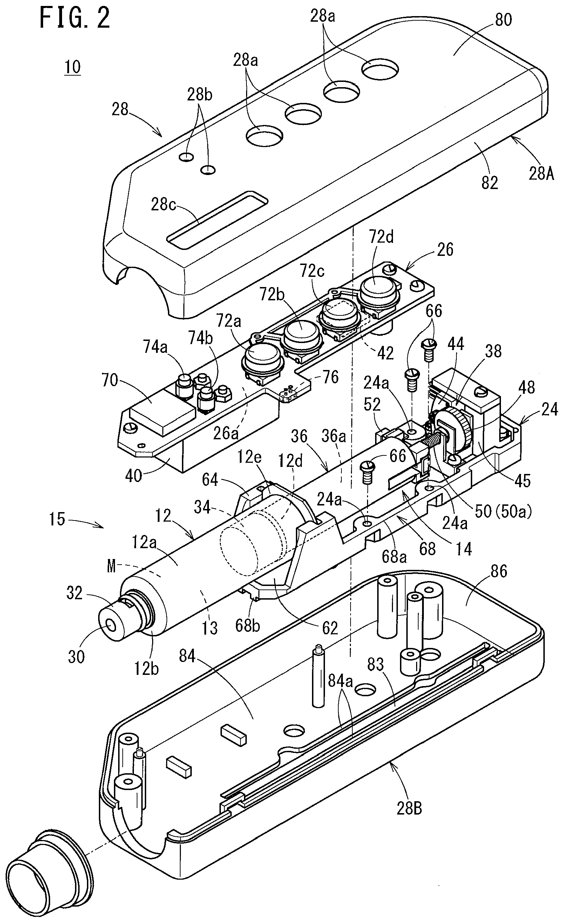

As illustrated in Fig. 2, the liquid medication administration device 10 includes the cylindrical body 12 filled with the liquid medication M, the plunger mechanism 14 that pushes the liquid medication M out of the cylindrical body 12, a chassis member 24 that supports the cylindrical body 12 and the plunger mechanism 14, a board 26 equipped with a control unit 42 (microcomputer) that controls a motor 44 and a battery 40 that supplies power required for operating the liquid medication administration device 10, and a housing 28 that houses these members.

The cylindrical body 12 has a hollow cylindrical shape, and the inner side of the cylindrical body 12 includes a liquid medication chamber 13. Specifically, the cylindrical body 12 includes a barrel 12a, a shoulder 12b, and a distal end 12c. The barrel 12a has an inner diameter and an outer diameter constant in an axial direction and has a proximal end opened. The shoulder 12b has an inner diameter and an outer diameter tapered in a distal direction from a distal end of the barrel 12a. The distal end 12c protrudes from the shoulder 12b in the distal direction. Being engaged with the chassis member 24 at a flange 12e, the cylindrical body 12 is fixed to the chassis member 24. The flange 12e is a first supported portion supported by the chassis member 24. The shoulder 12b may be the first supported portion supported by the chassis member 24.

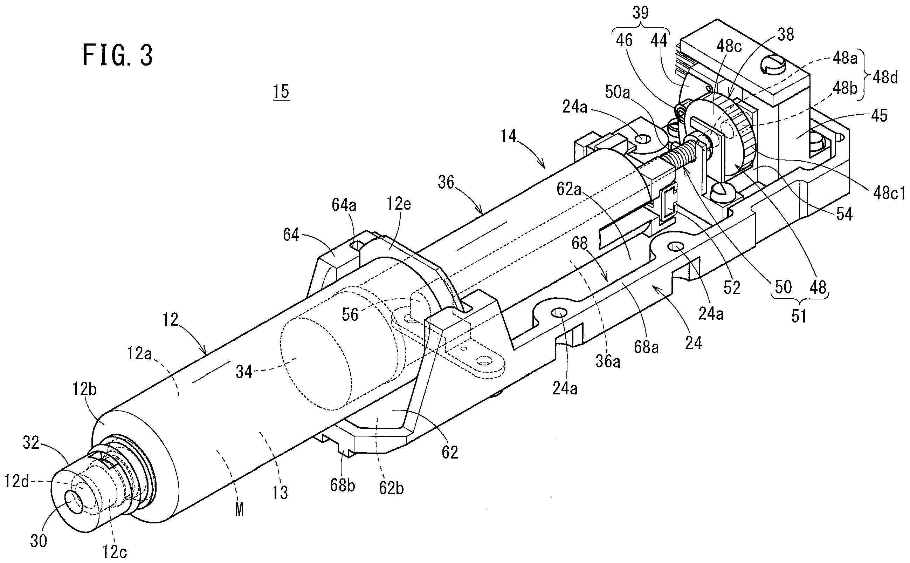

A liquid medication discharge port 12d (see Fig. 3) communicated with the liquid medication chamber 13 is formed at the distal end 12c. The liquid medication M is prefilled in the cylindrical body 12. The liquid medication discharge port 12d is sealed in a liquid-tight manner by a sealing member 30 that includes an elastic resin material such as a rubber material or an elastomer material. When the connector 18 illustrated in Fig. 1 is connected to the distal end 12c, the sealing member 30 is punctured by a needle 18a disposed in the connector 18. The sealing member 30 is fixed to the distal end 12c of the cylindrical body 12 by a cap 32 having a distal end provided with an opening. A distal surface of the sealing member 30 is exposed from the opening of the cap 32.

As illustrated in Fig. 3, the plunger mechanism 14 includes a gasket 34 slidably disposed inside the cylindrical body 12, a plunger body 36 connected to the gasket 34 and movable in the axial direction with respect to the cylindrical body 12, an advancing mechanism 38 that advances the plunger body 36 in the distal direction, and a drive mechanism 39 that drives the advancing mechanism 38 to advance the plunger body 36 in the distal direction. The plunger mechanism 14 advances the plunger body 36 connected to the gasket 34 by the advancing mechanism 38 and pushes the liquid medication M out of the cylindrical body 12.

The gasket 34 includes an elastic resin material such as a rubber material or an elastomer material. An outer periphery of the gasket 34 closely contacts an inner periphery of the cylindrical body 12 (barrel 12a) in a liquid-tight manner so as to close a proximal end of the liquid medication chamber 13 in a liquid-tight manner.

The plunger body 36 is a member movable in the axial direction to push out the liquid medication M. The gasket 34 is connected to a distal end of the plunger body 36. The plunger body 36 includes a hollow portion 36a opened downward and in a proximal direction.

The drive mechanism 39 includes the motor 44 (for example, a geared motor) that is driven under control of the control unit 42 (Fig. 2) using the battery 40 (Fig. 2) as a power supply, and includes a pinion 46, or a driving gear that is fixed to an output shaft of the motor 44. The advancing mechanism 38 includes a large gear 48, or a driven gear that meshes with the pinion 46, a feed screw shaft 50 engaged with the large gear 48 to transmit torque thereto, a nut member 52 screwed into the feed screw shaft 50, and a guide rail 60 that supports the nut member 52.

The motor 44 is fixed to the chassis member 24. The large gear 48 is larger in diameter than the pinion 46, being parallel to the pinion 46. The motor 44 may be fixed to the housing 28.

The large gear 48 includes a first shaft 48a and a second shaft 48b protruding in opposite directions along the axial direction. Specifically, the large gear 48 includes a gear body 48c and a rotary shaft 48d. The gear body 48c has an outer periphery provided with a plurality of teeth 48c1. The rotary shaft 48d is disposed in the rotation center of the gear body 48c, protruding from the gear body 48c in both the distal direction and the opposite direction, or the proximal direction. The first shaft 48a and the second shaft 48b are included in the rotary shaft 48d. A proximal end of the second shaft 48b is a second supported portion supported by the chassis member 24. A proximal surface of a portion protruding from an outer periphery of a proximal end of the feed screw shaft 50, a proximal surface of a portion protruding from an outer periphery of the rotary shaft 48d, or a proximal surface of the gear body 48c of the large gear 48 may be the second supported portion supported by the chassis member 24. Furthermore, the first shaft 48a and the second shaft 48b may be a single shaft member inserted into a hole formed in the center of the large gear 48.

The first shaft 48a and the second shaft 48b are rotatably supported by a support member 54 fixed to the chassis member 24. An end of the second shaft 48b penetrates the support member 54 and contacts a contact member 45 that is fixed to a base plate 62, to be described later, of the chassis member 24. Accordingly, in the large gear 48, the axial load is supported by the contact member 45. An engagement projection having a non-circular cross-sectional shape disposed at a distal end of the first shaft 48a is inserted into an engagement recess having a non-circular cross-sectional shape disposed at the proximal end of the feed screw shaft 50. Accordingly, the large gear 48 and the feed screw shaft 50 are engaged with each other and enable transmission of torque from the large gear 48 to the feed screw shaft 50. The large gear 48 and the feed screw shaft 50 are included in a feed screw 51. The large gear 48 may be provided in an integrated manner with the feed screw shaft 50. Alternatively, the feed screw shaft 50 may be inserted into a hole disposed in the large gear 48, and the feed screw shaft 50 may be directly fixed to the large gear 48.

The feed screw shaft 50 is disposed along an axis of the cylindrical body 12. An outer periphery of the feed screw shaft 50 is provided with an external thread 50a over a predetermined range in the axial direction. In the initial state illustrated in Fig. 3, at least a part of the feed screw shaft 50 in the axial direction is disposed inside the plunger body 36 (within the hollow portion 36a). The proximal end of the feed screw shaft 50 is rotatably supported by the support member 54. A distal end of the feed screw shaft 50 is rotatably supported by a distal end support member 56 that is fixed to the chassis member 24.

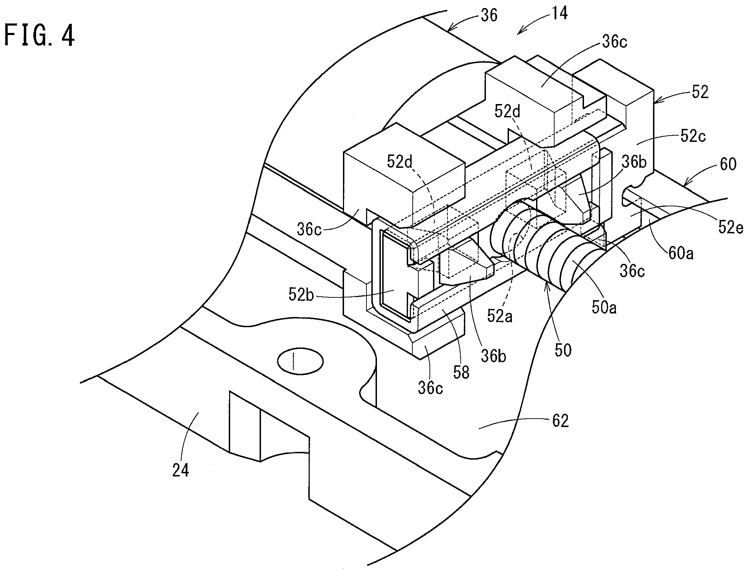

In Fig. 4, the nut member 52 is a female screw that includes, for example, a hard resin material and includes an internal thread 52a (a screw hole) into which the feed screw shaft 50 is screwed. Along with the rotation of the feed screw shaft 50, the nut member 52 moves in the axial direction. Specifically, the nut member 52 includes a nut body 52b provided with the internal thread 52a and includes a slide 52c formed at one end of the nut body 52b. The nut member 52 may be omitted, and the internal thread 52a and the slide 52c may be disposed on the plunger body 36.

The nut body 52b is provided with a pair of holes 52d penetrating the nut body 52b in the axial direction. Claws 36b formed at a proximal end of the plunger body 36 are engaged with the pair of holes 52d. A reinforcement cover 58 that includes, for example, a metallic material is mounted on an outer surface of the nut body 52b. The reinforcement cover 58 is held by a plurality of holding units 36c disposed at the proximal end of the plunger body 36.

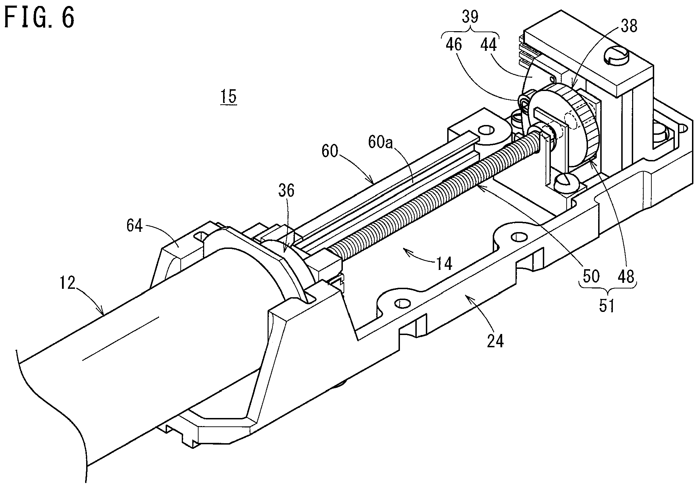

The slide 52c is supported by the guide rail 60 (see also Fig. 6) which is fixed to the chassis member 24 and extends parallel to the feed screw shaft 50 so as to be slidable in the axial direction. Accordingly, along with the rotation of the feed screw shaft 50, the nut member 52 is smoothly movable in the axial direction of the plunger mechanism 14. Specifically, the slide 52c includes a guide protrusion 52e protruding toward the guide rail 60. The guide protrusion 52e is inserted into a guide groove 60a formed in the guide rail 60. The guide groove 60a extends in the axial direction of the plunger mechanism 14.

As illustrated in Fig. 3, the chassis member 24 includes the base plate 62, a flange mounting portion 64, and the contact member 45. The base plate 62 extends along an inner surface of the housing 28 (a lower housing 28B to be described later) (Fig. 2). The flange mounting portion 64 to which the flange 12e is attached protrudes in a thickness direction of the base plate 62 (upward) from an upper surface 62a of the base plate 62. The contact member 45 is attached to the upper surface 62a of the base plate 62. The flange mounting portion 64 is a first supporting portion that supports at least a distal surface of the first supported portion (flange 12e) of the cylindrical body 12. As another mode of the first supporting portion, the chassis member 24 may be provided with a portion that supports a distal surface of the shoulder 12b of the cylindrical body 12.

The contact member 45 is provided with a second supporting portion that supports a proximal surface of the second supported portion (the proximal end of the second shaft 48b) of the advancing mechanism 38. The contact member 45 is preferably a metallic L-shaped plate. Accordingly, it is possible to support the proximal surface of the second supported portion (the proximal end of the second shaft 48b) of the advancing mechanism 38 more reliably. The second supporting portion may be disposed in an integrated manner with the chassis member 24. As another mode of the second supporting portion, the chassis member 24 may be provided with a portion that supports the proximal surface of the portion protruding from the outer periphery of the proximal end of the feed screw shaft 50, the proximal surface of the portion protruding from the outer periphery of the rotary shaft 48d, or the proximal surface of the gear body 48c of the large gear 48.

The chassis member 24 has an elongated shape along an axis of the plunger mechanism 14 (along the feed screw shaft 50). The cylindrical body 12, the plunger mechanism 14, and the chassis member 24 are included in a liquid feeding unit 15. The chassis member 24 includes, for example, a hard resin and a metallic material. As illustrated in Fig. 2, the chassis member 24 is fixed to the housing 28 (lower housing 28B) by a plurality of fastening components 66 (screws in the illustrated example). A plurality of screw holes 24a into which the screws are inserted penetrates the chassis member 24 in a thickness direction of the chassis member 24.

The base plate 62 includes opposing plate surfaces (the upper surface 62a and a lower surface 62b). The plunger mechanism 14 is disposed on the upper surface 62a of the base plate 62. The motor 44 and the contact member 45 of the plunger mechanism 14 are fixed to a proximal end of the base plate 62. The guide rail 60 is fixed along one oval of the base plate 62. The lower surface 62b of the base plate 62 faces the lower housing 28B.

The flange mounting portion 64 is disposed in an integrated manner with the base plate 62 at a position near a distal end of the base plate 62. The flange mounting portion 64 includes a U-shaped flange holding groove 64a into which the flange 12e is inserted. The cylindrical body 12 is fixed to the chassis member 24 when the flange 12e fits into the flange holding groove 64a.

The chassis member 24 further includes a rib structure 68 that prevents deformation of the chassis member 24. The rib structure 68 protrudes in the thickness direction of the base plate 62 and extends along a movable direction of the plunger body 36 (the axial direction of the pressing mechanism). The rib structure 68 includes an edge rib 68a and lower surface ribs 68b. The edge rib 68a is disposed at an edge of the base plate 62 (specifically, the other long side of the base plate 62), protruding upward from the upper surface 62a of the base plate 62. The lower surface ribs 68b protrude downward from the lower surface 62b of base plate 62.

The edge rib 68a is continuous with the flange mounting portion 64. A height of the edge rib 68a protruding from the base plate 62 is lower than a height of the flange mounting portion 64 protruding from the base plate 62. At least one of the plurality of screw holes 24a is formed in the edge rib 68a. The edge rib 68a may be disposed also at an edge on one long side 63a of the base plate 62.

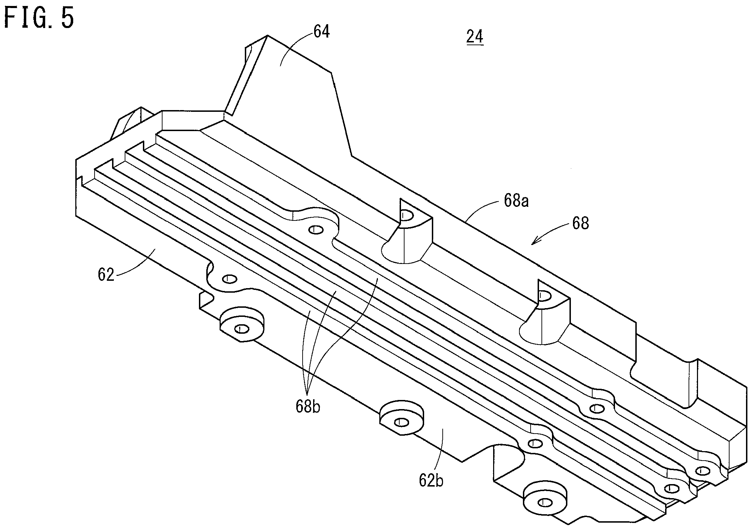

As illustrated in Fig. 5, a plurality of lower surface ribs 68b is disposed parallel to each other along a longitudinal direction of the chassis member 24. The lower surface ribs 68b are formed over the entire length of the base plate 62.

As illustrated in Fig. 2, in addition to the battery 40 and the control unit 42, a plurality of buttons 72a to 72d for various operations, a plurality of light emitting units 74a and 74b, and a speaker 70 are mounted on the board 26. As illustrated in Figs. 1 and 2, the plurality of buttons 72a to 72d include a power button 72a, a priming button 72b, a liquid feeding button 72c, and a pause button 72d. The buttons 72a to 72d are exposed to the outside of the housing 28 through button exposure holes 28a formed in the housing 28.

The plurality of light emitting units 74a and 74b include a first light emitting unit 74a and a second light emitting unit 74b that emit different colors. The first light emitting unit 74a emits different colors, used for notifying the operation state of the liquid medication administration device 10. The second light emitting unit 74b lights up or blinks to notify the occurrence of an error. The first light emitting unit 74a and the second light emitting unit 74b include, for example, LEDs. The first light emitting unit 74a and the second light emitting unit 74b are exposed to the outside of the housing 28 through light emitting unit exposure holes 28b formed in the housing 28.

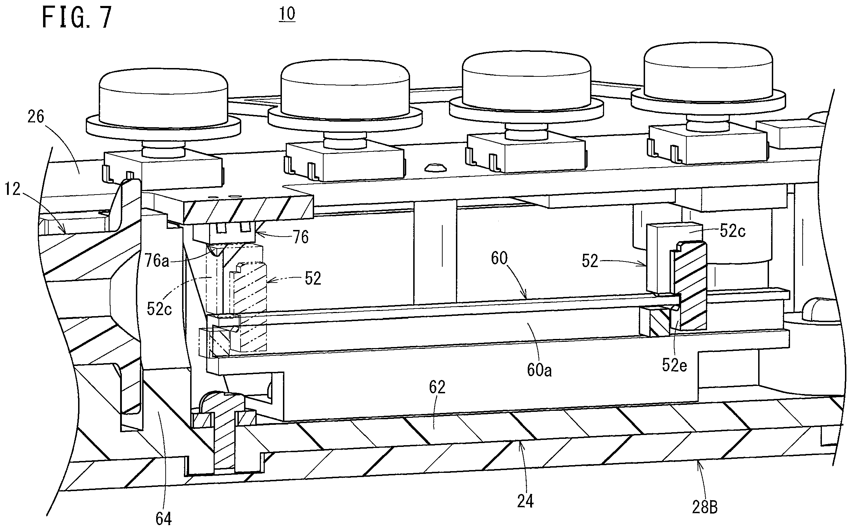

The board 26 is further provided with a switch 76 for detecting completion of liquid feeding (completion of administration) of the liquidmedicationM. Specifically, the switch 76 is attached to the lower surface 26a of the board 26 that faces the chassis member 24. The switch 76 is arranged and disposed in such a manner that the switch 76 is pushed by the slide 52c of the nut member 52 when the plunger body 36 moves to an administration completion position of the liquid medication M (Fig. 7).

The housing 28 illustrated in Fig. 2 houses the cylindrical body 12 and the plunger mechanism 14 and supports the chassis member 24. The housing 28 includes an upper housing 28A and the lower housing 28B. The upper housing 28A and the lower housing 28B have a shape obtained by dividing the housing 28 in a thickness direction of the housing 28. The housing 28 includes a chassis supporting portion 83 which extends along at least the lower surface 62b of the base plate 62 and which supports the base plate 62.

The upper housing 28A includes a flat upper wall 80 and an upper peripheral wall 82 protruding downward from a peripheral edge of the upper wall 80. The upper wall 80 is provided with a residual liquid observation window 28c as well as the button exposure holes 28a and the light emitting unit exposure holes 28b. The residual liquid observation window 28c is used for checking a residual quantity of the liquid medication M filled in the cylindrical body 12.

The lower housing 28B includes a flat bottom wall 84 and a lower peripheral wall 86 protruding upward from a peripheral edge of the bottom wall 84. The bottom wall 84 is provided with a plurality of rib disposition grooves 84a (slits) into which the lower surface ribs 68b of the chassis member 24 are inserted. The rib disposition grooves 84a are arranged in a plurality of rows along a longitudinal direction of the lower housing 28B. The chassis supporting portion 83 is included in a part of the bottom wall 84. The rib disposition grooves 84a are formed in the chassis supporting portion 83.

Hereinafter described is the operation of the liquid medication administration device 10 having the aforementioned arrangement.

When the liquid medication administration device 10 illustrated in Fig. 1 is in use, the liquid medication administration device 10 is taken out of a cold storage, allowed to stand at normal temperature, and brought back to room temperature. Next, the surface (distal surface) of the sealing member 30 which is to be connected with the connector 18 is wiped off with a alcohol pad or the like so as to be disinfected. Next, the administration line 16 (for example, the needle tube 17) is connected to the liquid medication administration device 10.

Next, when the power button 72a is pressed (pressed and held) for a predetermined time or more, the first light emitting unit 74a lights up in a first color, the speaker 70 (Fig. 2) plays the starting sound, and the device is powered on. Next, when the priming button 72b is pressed continuously, the first light emitting unit 74a blinks in the first color, leading to priming (filling of the administration line 16 with the liquid medication M). In this case, it is possible to see the completion of priming by visually checking a drop of the liquid medication M from a needle tip 21a.

The liquid medication administration device 10 is then attached to a patient by being stuck to the skin S or mounted on clothes of the patient. Next, the skin S is punctured with the needle 21. The liquid medication administration device 10 may be attached to the patient before puncture of the skin S with the needle 21.

When the liquid feeding button 72c is pressed for a predetermined time or more, the first light emitting unit 74a blinks in a second color (a color different from the first color), leading to liquid feeding (administration of the liquid medication M).

Specifically, when the control unit 42 determines that the liquid feeding is started along with the operation of the liquid feeding button 72c, the motor 44 illustrated in Fig. 3 is driven to transmit torque from the pinion 46 to the large gear 48. Along with the rotation of the large gear 48, the torque is transmitted to the feed screw shaft 50 that is engaged with the large gear 48. Along with the rotation of the feed screw shaft 50, the nut member 52 screwed into the feed screw shaft 50 advances and the plunger body 36 is pushed and advanced as illustrated in Fig. 6. Accordingly, the liquid medication M is pushed out of the cylindrical body 12. The liquid medication M pushed out of the cylindrical body 12 is administered (injected) to the patient's body through the administration line 16 (Fig. 1) inserted into the patient.

When the liquid feeding is completed by the plunger body 36 having advanced to a predetermined position, a sound indicating the completion of liquid feeding is output from the speaker 70 illustrated in Fig. 2, and the first light emitting unit 74a lights in the first color. The completion of liquid feeding is detected by the switch 76 disposed on the board 26. As illustrated in Fig. 7, in the initial state before the start of liquid feeding, the nut member 52 is located at a position apart from the switch 76 in the proximal direction. As illustrated by imaginary lines in Fig. 7, when the nut member 52 advances for liquid feeding and the plunger body 36 moves to a liquid feeding completion position, a detective projection of the switch 76 is pressed by the nut member 52. Accordingly, the completion of liquid feeding is detected.

On completion of liquid feeding, the needle 21 is drawn from the skin S (under the skin). Then, the liquid medication administration device 10 is discarded.

In this case, the liquid medication administration device 10 according to this embodiment has the following effects.

According to the liquid medication administration device 10, as illustrated in Fig. 2, the chassis member 24 integrates the cylindrical body 12 and the plunger mechanism 14, and the chassis member 24 is fixed to the housing 28. With such an arrangement, the housing 28 is reinforced by the chassis member 24, and a dual structure formed by the chassis member 24 and the housing 28 prevents deformation (warpage) of the housing 28 when the liquid medication M is pushed out. Accordingly, an axis of the advancing mechanism 38 is prevented from being inclined relative to the axis of the cylindrical body 12, which enables administration of the liquid medication M at a desired liquid feeding speed (constant speed). It is preferable that the contact member 45 of the chassis member 24 should be a metallic L-shaped plate attached to the upper surface 62a of the base plate 62. With such an arrangement, it is possible to support the proximal surface of the second supported portion (the proximal end of the second shaft 48b) of the advancing mechanism 38 more reliably and to prevent deformation (warpage) of the housing 28 more reliably when the liquid medication M is pushed out.

The chassis member 24 includes the rib structure 68 protruding from the base plate 62 and extending along the axial direction of the cylindrical body 12. This arrangement efficiently enhances rigidity of the chassis member 24 and prevents warpage of the chassis member 24 itself.

The rib structure 68 extends at least from a portion near the first supporting portion (the flange mounting portion 64) to a portion near the second supporting portion (the contact member 45). This arrangement more efficiently prevents the chassis member 24 from being deformed in a direction in which the axis of the advancing mechanism 38 inclines relative to the axis of the cylindrical body 12.

The rib structure 68 includes the edge rib 68a disposed at the edge of the base plate 62 along the axial direction of the cylindrical body 12. This arrangement enables the rib structure 68 to be disposed without interfering with the plunger mechanism 14.

The rib structure 68 is continuous with the first supporting portion (flange mounting portion 64). In this arrangement, the first supporting portion is continuous with the rib structure 68, which efficiently enhances rigidity of the chassis member 24.

The rib structure 68 includes the lower surface ribs 68b protruding from the lower surface 62b of the base plate 62. This arrangement efficiently enhances rigidity of the chassis member 24 while avoiding an interference between the advancing mechanism 38 and the rib structure 68.

The chassis supporting portion 83 includes the rib disposition grooves 84a into which the lower surface ribs 68b are inserted. This arrangement enables positioning of the chassis member 24 relative to the housing 28 before fixing the chassis member by the fastening member. Furthermore, the lower surface ribs 68b are inserted into the rib disposition grooves 84a, which reduces the device in thickness.

The rib structure 68 and the first supporting portion are formed in an integrated manner with the base plate 62. With this arrangement, the chassis member 24 is less likely to warp.

The cylindrical body 12 includes the barrel 12a filled with the liquid medication M and the flange 12e protruding outward from the proximal end of the barrel 12a. The first supporting portion supports the distal surface of the flange 12e and a proximal surface of the flange 12e. This arrangement enables independent positioning of the cylindrical body 12 on the chassis member 24, which facilitates assembly.

The second supporting portion is disposed on the metallic contact member 45 attached to the base plate 62. Making the contact member 45 from metal prevents the second supporting portion itself from bending and being unable to support the second supported portion.

The advancing mechanism 38 includes the feed screw 51, the female screw, and the guide rail 60. The feed screw 51 extends along the axial direction of the cylindrical body 12 and has an outer periphery provided with the external thread 50a. The female screw is disposed on the plunger body 36 and has an inner periphery provided with the internal thread 52a that is screwed into the external thread 50a. The guide rail 60 extends along the axial direction of the cylindrical body 12 and supports the female screw. The female screw is supported by the guide rail 60, being non-rotatable with respect to the chassis member 24 and movable with respect to the chassis member 24 along the axial direction of the cylindrical body 12. The feed screw 51 is rotatably supported by the chassis member 24. The drive mechanism 39 causes the feed screw 51 to rotate. When the feed screw 51 rotates, the female screw advances in the distal direction with respect to the feed screw 51. The feed screw 51 extends in the axial direction, which increases the loss of force when the housing 28 is warped. Therefore, it is more important to prevent warpage.

The present invention is not limited to the embodiment described above and may be modified in various manners without departing from the gist of the present invention.

## Claims

1. A liquid medication administration device (10) for administering a liquidmedication (M) to a living body, the liquid medication administration device (10) comprising:
a cylindrical body (12) filled with the liquid medication (M) ;
a gasket (34) slidably disposed inside the cylindrical body (12) ;
a plunger body (36) that pushes the gasket (34) in a distal direction to push the liquidmedication (M) out of the cylindrical body (12);
an advancing mechanism (38) that advances the plunger body (36) in the distal direction;
a drive mechanism (39) that drives the advancing mechanism (38) to advance the plunger body (36) in the distal direction;
a chassis member (24) that supports at least the cylindrical body (12) and the advancing mechanism (38); and
a housing (28) that houses the cylindrical body (12), the plunger body (36), the advancing mechanism (38), the drive mechanism (39), and the chassis member (24),
wherein the cylindrical body (12) includes a first supported portion supported by the chassis member (24),
the advancing mechanism (38) includes a second supported portion supported by the chassis member (24),
the chassis member (24) includes a base plate (62) that has a board shape and has an upper surface and a lower surface, a first supporting portion that protrudes from the upper surface of the base plate (62) and supports at least a distal surface of the first supported portion of the cylindrical body (12), and a second supporting portion that protrudes from the upper surface of the base plate (62) and supports at least a proximal surface of the second supported portion of the advancing mechanism (38), and
the housing (28) includes a chassis supporting portion (83) that extends at least along a lower surface of the base plate (62) and supports the base plate (62).

2. The liquid medication administration device (10) according to claim 1,
wherein the chassis member (24) includes a rib structure (68) protruding from the base plate (62) and extending along an axial direction of the cylindrical body (12).

3. The liquid medication administration device (10) according to claim 2,
wherein the rib structure (68) extends at least from a portion near the first supporting portion to a portion near the second supporting portion.

4. The liquid medication administration device (10) according to claim 2 or 3,
wherein the rib structure (68) includes an edge rib (68a) disposed at an edge of the base plate (62) along the axial direction of the cylindrical body (12).

5. The liquid medication administration device (10) according to claim 4,
wherein the edge rib (68a) includes a hole (24a), and
the chassis member (24) is fixed to the housing (28) by a fastening component (66) inserted into the hole (24a).

6. The liquid medication administration device (10) according to any one of claims 2 to 5,
wherein the rib structure (68) is continuous with the first supporting portion.

7. The liquid medication administration device (10) according to any one of claims 2 to 6,
wherein the rib structure (68) includes a lower surface rib (68b) protruding from the lower surface of the base plate (62) .

8. The liquid medication administration device (10) according to claim 7,
wherein the chassis supporting portion (83) includes a slit (84a) into which the lower surface rib (68b) is inserted.

9. The liquid medication administration device (10) according to any one of claims 2 to 8,
wherein the rib structure (68) and the first supporting portion are formed in an integrated manner with the base plate (62) .

10. The liquid medication administration device (10) according to any one of claims 1 to 9,
wherein the cylindrical body (12) includes a barrel (12a) filled with the liquid medication (M) and a flange (12e) protruding outward from a proximal end of the barrel (12a), and
the first supporting portion supports a distal surface of the flange (12e) and a proximal surface of the flange (12e).

11. The liquid medication administration device (10) according to any one of claims 1 to 10,
wherein the second supporting portion is disposed on a metallic contact member (45) attached to the base plate (62) .

12. The liquid medication administration device (10) according to any one of claims 1 to 11,
wherein the advancing mechanism (38) comprises:
a feed screw (51) extending along the axial direction of the cylindrical body (12) and having an outer periphery provided with an external thread (50a);
a female screw disposed on the plunger body (36) and having an inner periphery provided with an internal thread (52a) that is screwed into the external thread (50a); and
a guide rail (60) that extends along the axial direction of the cylindrical body (12) and supports the female screw;
wherein the female screw is supported by the guide rail (60), being non-rotatable with respect to the chassis member (24) and movable with respect to the chassis member (24) along the axial direction of the cylindrical body (12),
the feed screw (51) is rotatably supported by the chassis member (24),
the drive mechanism (39) is configured to rotate the feed screw (51), and
the female screw advances in the distal direction with respect to the feed screw (51) along with rotation of the feed screw (51).

13. The liquid medication administration device (10) according to claim 12,
wherein the drive mechanism (39) includes a motor (44) and a driving gear (46) fixed to an output shaft of the motor (44),
the feed screw (51) includes a feed screw shaft (50) extending along the axial direction of the cylindrical body (12) and including the external thread (50a) and includes a driven gear (48) connected to the feed screw shaft (50) in a non-rotatable manner and to which rotation of the driving gear (46) is transmitted.

14. The liquid medication administration device (10) according to claim 13,
wherein the driven gear (48) includes a gear body (48c) having an outer periphery provided with a plurality of teeth and includes a rotary shaft (48d) disposed at a rotation center of the gear body (48c) and protruding from the gear body (48c) in both the distal direction and the opposite direction, or a proximal direction,
wherein the rotary shaft (48d) has a distal end connected to a proximal end of the feed screw shaft (50), and the proximal end of the rotary shaft (48d) is supported by the second supporting portion.
